# EUROPEAN PATENT APPLICATION

(11) **EP 4 411 743 A1**
(43) Date of publication of application: **07.08.2024**
(21) Application number: 23175572.9
(22) Date of filing: 26.05.2023
(51) Int. Cl.: G16H 10/60

(54) **PATIENT REGISTRY SYSTEM**

(30) Priority: 02.02.2023 US 202363442849 P; 08.05.2023 US 202318313614
(71) Applicant: Corbishley, Brian, Middletown, DE 19709 (US)
(72) Inventor: Corbishley, Brian, Middletown, DE 19709 (US)
(74) Representative: Abel & Imray LLP

(57) **Abstract**

A portable device for storing and providing access to a user's medical and demographic information. The portable storage device allow a medical provider immediate access to the user's current medical, demographic, and insurance information without the need to ask questions or fill out written forms. A system and method of using the portable storage device includes a customer portal hosted on a remote server that is accessible to the user. The user can create and update their electronic user record on the portal as needed. The medical provider can access the electronic user record via an external device interface at the point of service. The external device interface may communicate with a local electronic medical record system and may update the electronic user record on the portable storage device following an appointment.

## Description

### FIELD OF THE INVENTION

The present invention generally relates to the portability of electronic medical records. More specifically, the present invention relates to a system and method for retaining individual medical records and electronically retrieving the medical records at a point of service. Accordingly, the present specification makes specific reference thereto. However, it is to be appreciated that aspects of the present invention are also equally amenable to other like applications, devices and methods of manufacture.

### BACKGROUND

People who visit a medical provider's office, clinic, hospital, or other health care facility are typically required to provide their past medical history prior to treatment. They are also typically required to provide their insurance and billing information for every visit. This process must be continuously repeated, often by hand using paper forms, if a person visits several different doctors. Constantly filling out paperwork for every visit or appointment can be frustrating and time consuming for patients.

An electronic health record is an electronic version of a patient's medical history that is maintained by a provider or a health network over time. These electronic records may include administrative clinical data relevant to that persons care under a variety of medical providers. The data may include demographics, insurance information, billing information, progress notes, problems, medications, allergies, vital signs, past medical history, immunizations, clinical reports, and the like. These records function essentially as a digitized version of a patient's chart. These records may be shared or accessed through existing networks or exchanges as long as the data is collected through the network.

Electronic health record systems are designed to store data accurately and capture the condition of a patient across time. These systems eliminate the need to physically find and access a patient's previous hard copy medical records and assists in ensuring that the data is current. These systems also allow for better open communication between the patient and the medical provider in a secure and protected manner. These systems further eliminate the need for data replication as they only require a single modifiable file for each patient. This keeps files current while decreasing the risk of lost paperwork and improving efficiency as long as the patient remains in that particular system.

Many larger health care organization systems have individualized versions of electronical medical record systems that allow everyone in that particular network to access the information as long as they have permissions. Unfortunately, each electronical medical record system is different and there is generally no interoperability or accessibility between different healthcare systems. If a patient attempts to receive medical service via a medical provider that is not in that network, there is no easy way for either the provider or the patient to access those electronic medical records from that network.

Therefore, there exists a long felt need in the art for a way for individuals themselves to have real-time and continuous access to their own medical information. There is also a long felt need in the art for a portable information storage device that allows providers access to the medical information at any medical provider's office, clinic, hospital, or other health care facility. Additionally, there is a long felt need in the art for a way to streamline a medical check in process and eliminate the need to fill out paperwork by hand at each visit. Finally, there is a long felt need in the art for a system that allows the user or provider to update information on a portable device.

In this manner, the improved system of the present invention accomplishes all of the foregoing objectives, thereby providing an easy solution to allow individuals to have real-time and continuous access to their medical information. A primary feature of the present invention is a portable device that stores the medical information that is accessible at any medical provider's office, clinic, hospital, or other health care facility. The device and system help expedite medical care check in processes, thereby eliminating the need to fill out paperwork by hand each time a new doctor's visit occurs. The device is configured to interact with an input at the point of service so that the user simply swipes or uploads their card into a system that contains all necessary personal and insurance information, ensuring the new doctor can obtain the information quickly. Finally, the improved device and system of the present invention can enable patients to update information on the card should anything change regarding medical or insurance information.

### SUMMARY

The following presents a simplified summary in order to provide a basic understanding of some aspects of the disclosed innovation. This summary is not an extensive overview, and it is not intended to identify key/critical elements or to delineate the scope thereof. Its sole purpose is to present some concepts in a simplified form as a prelude to the more detailed description that is presented later.

The subject matter disclosed and claimed herein, in one embodiment thereof, comprises an information storage device. The information storage device is configured to retain an electronic user record. The electronic user record includes a user's medical data and a user's demographic data. The information storage device is portable and updatable.

The information storage device comprises a memory, a communication module, and a processor in communication with the memory and communication module. The memory is a non-transitory computer readable memory configured to store the electronic user record. The communication module communicates the stored electronic user record at a point of service. The stored electronic user record may be accessed wirelessly, magnetically, or via a connection interface.

The subject matter disclosed and claimed herein, in one embodiment thereof, comprises a system for managing an electronic user record. The system comprises a computer server, a plurality of portable information storage devices, and a plurality of external device interfaces. The system is configured to allow the user to have a portable electronic user record accessible at a point of service that can be updated as required.

In embodiments of the invention, the information storage device further comprises a data port. In embodiments of the invention, the point of service is a medical facility, an imaging provider, a laboratory, a medical provider's office, a rehabilitation facility, a pharmacy, or a long term care facility.

The computer server comprises a memory and a processor. The memory stores a set of computer readable software instructions configured as a customer portal. The customer portal is remotely accessible to the user over a network via a user access device. The user uploads the user's medical and demographic data to the customer portal to create an electronic user record. The user may update the electronic user record over the network via the user access device as needed. The electronic user record is then downloadable to one of the portable information storage devices.

In embodiments of the invention, the user access device is a smart phone, a laptop, a personal computer, or a personal digital assistant. In embodiments of the invention, the user's medical record includes the user's medical history, medical providers, immunizations, medications, medical test results, medical study results, medical imaging, diagnoses, symptoms, health complaints, or medical procedures. In embodiments of the invention, the user's demographic record includes the user's contact information, insurance information, and non-medical personal information.

Each portable information storage device comprises a memory, a communication module, and a processor in communication with the memory and communication module. The memory is a non-transitory computer readable memory configured to store the electronic user record on the portable information storage device. The communication module communicates the stored electronic user record at a point of service. The stored electronic user record may be accessed wirelessly, magnetically, or via a connection interface.

Each external device interface is configured to communicate with each portable information storage device and access the stored electronic user record. Each external device interface may then access an electronic medical record system at the point of service and communicate the stored electronic user record to the electronic medical record system. New medical or demographic data may then be transferred from the electronic medical record system to the portable information storage device via the external device interface.

The subject matter disclosed and claimed herein, in one embodiment thereof, comprises a method for creating and administering a user's electronic user record. The user accesses a customer portal stored on a computer-based system over a network using a user access device and constructs an electronic user record on a customer portal. The user uploads a user's medical data and a user's demographic data to the customer portal into the electronic user record. The user's electronic user record is then further stored on a portable information storage device.

The user's electronic user record is accessible at a point of service via an external device interface. The external device interface can upload the electronic user record to an electronic medical records system of the point of service. Then the external device interface can modify the electronic user record with any new user medical or demographic data. Alternatively, the user may update the electronic user record via the customer portal on the computer-based system. Then the updated electronic user record is then updated on the portable information storage device.

To the accomplishment of the foregoing and related ends, certain illustrative aspects of the disclosed innovation are described herein in connection with the following description and the annexed drawings. These aspects are indicative, however, of but a few of the various ways in which the principles disclosed herein can be employed and is intended to include all such aspects and their equivalents. Other advantages and novel features will become apparent from the following detailed description when considered in conjunction with the drawings.

### BRIEF DESCRIPTION OF THE DRAWINGS

The description refers to provided drawings in which similar reference characters refer to similar parts throughout the different views, and in which:
FIG. 1 illustrates a front perspective view of one embodiment of an information storage device of the present invention for storing an electronic user record in accordance with the disclosed architecture.
FIG. 2 illustrates a rear perspective view of the information storage device of the present invention for storing an electronic user record in accordance with the disclosed architecture.
FIG. 3 illustrates a front perspective view of the information storage device of the present invention for storing an electronic user record in accordance with the disclosed architecture.
FIG. 4 illustrates a diagram illustrating hardware and software components of the information storage device of the present invention for storing an electronic user record in accordance with the disclosed architecture.
FIG. 5 illustrates an exemplary electronic user record storable and modifiable on the information storage device of the present invention in accordance with the disclosed architecture.
FIG. 6 illustrates a diagram of a system for managing an electronic user record of the present invention in accordance with the disclosed architecture.
FIG. 7 illustrates a flowchart of a method for creating and administering an electronic user record on a computer-based system accessible via a portable information storage device connected to the computer-based system over a network of the present invention in accordance with the disclosed architecture.

### DETAILED DESCRIPTION

The innovation is now described with reference to the drawings, wherein like reference numerals are used to refer to like elements throughout. In the following description, for purposes of explanation, numerous specific details are set forth in order to provide a thorough understanding thereof. It may be evident, however, that the innovation can be practiced without these specific details. In other instances, well-known structures and devices are shown in block diagram form in order to facilitate a description thereof. Various embodiments are discussed hereinafter. It should be noted that the figures are described only to facilitate the description of the embodiments. They do not intend as an exhaustive description of the invention or do not limit the scope of the invention. Additionally, an illustrated embodiment need not have all the aspects or advantages shown. Thus, in other embodiments, any of the features described herein from different embodiments may be combined.

The present invention, in one exemplary embodiment, is a patient registry system. The unique information system comprised of software, hardware, and a related mobile application for the purpose of expediting the medical care check in process. With this system, the patient will have a card or other portable information storage device that contains all necessary personal and insurance information, and the facility will only need to swipe the card during the check in interaction. The patient will have the ability to update the information on the card as necessary.

The card could be similar to a credit card but would have a chip or magnetic storage medium that stores the patient's medical history. A card reader would be relatively small with a slot or wireless communication for accessing the information on the card. Hospitals and doctor's offices would have software allowing the reader to identify and store the data of each individual patient.

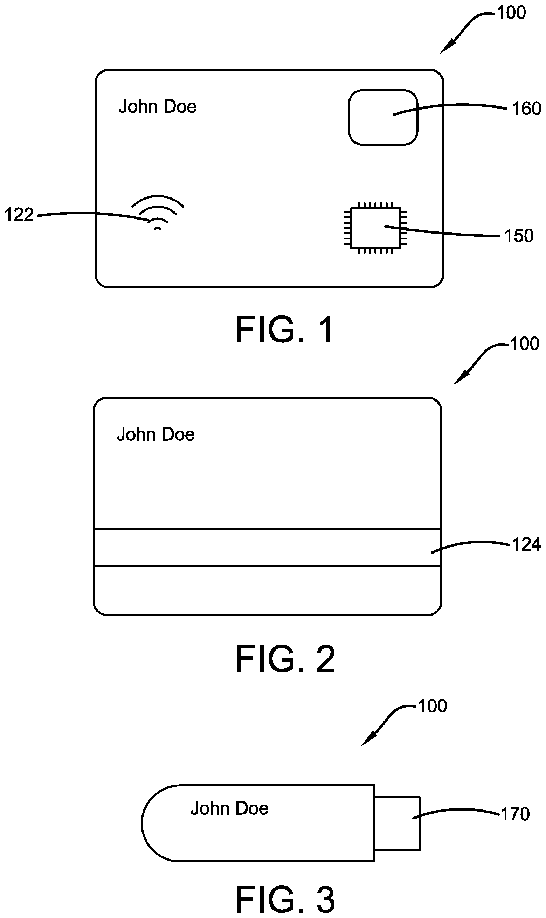

Referring initially to the drawings, FIGS. 1-5 illustrate an information storage device 100. The information storage device 100 is designed to allow a user to always have immediate access to their medical information. The user can bring the information storage device 100 to a doctor's office visit or any kind of medical appointment and avoid the need to manually update the records at a point of service. The information storage device 100 can interact with any point of service electronic medical record system to update the system without the need for manual inputs, thereby saving time and improving efficiency in maintaining an accurate up to date medical record.

As illustrated in FIGS. 1-3, the information storage device 100 is a portable device that is easily carried by the user for access at the point of service. The point of service may include a medical facility, an imaging provider, a laboratory, a medical provider's office, a dental office, a rehabilitation facility, a pharmacy, a therapist's office, a long term care facility, or any other location that may necessitate access to the user's medical record or related demographic information. The information storage device 100 is configured to retain an electronic user record 140 of the user, their family, or their household. The information storage device 100 may be configured as an integrated circuit card, a magnetic strip card, a memory stick, an external hard drive, a portable solid state drive, a flash drive, or any other portable electronic storage device that is configured to retain electronic data.

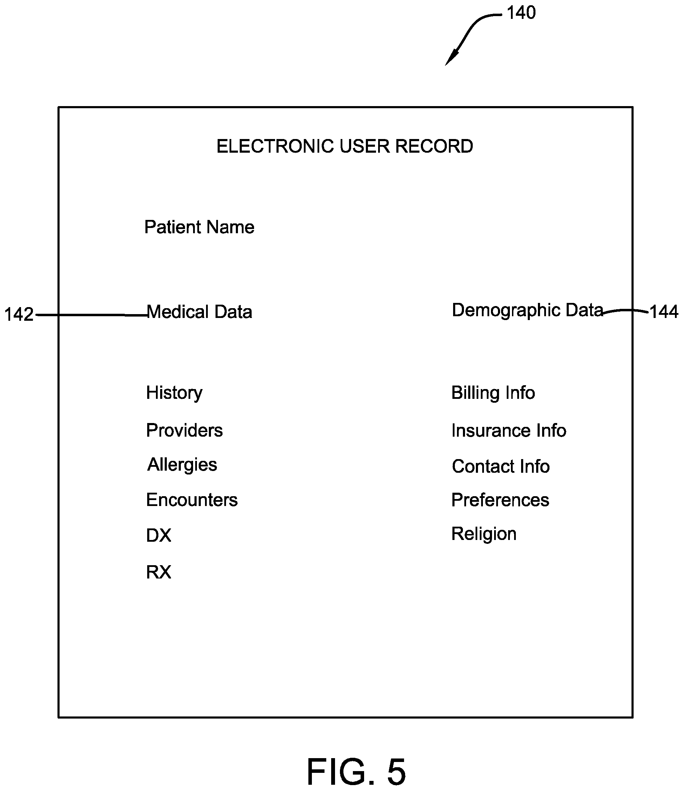

As illustrated in FIG. 5, the electronic user record 140 may include a user's medical data 142 and a user's demographic data 144. The user's medical data 142 can include, but is not limited to, the user's medical history, medical providers, immunization history, medications (current and past), medical test results, medical study results, medical imaging, diagnoses, symptoms, health complaints, medical procedures, living wills, do not resuscitate orders, advanced directives, and the like. The user's demographic data 144 may include, but is not limited to, the user's contact information, insurance information, non-medical personal information, next of kin information, emergency contacts, legal information, a health care power of attorney, and the like. The electronic user record 140 is updatable as discussed *infra.*

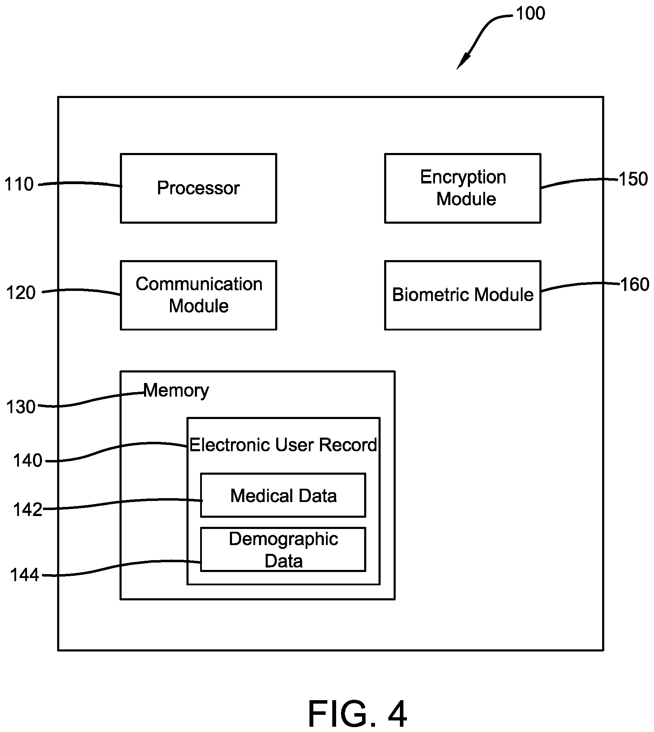

As illustrated in FIG. 4. the information storage device 100 comprises a memory 130, a communication module 120, and a processor 110. The processor 110 is in communication with the memory 130 and communication module 120. The processor 110 may be any data processing logic and control integrated circuit or chip. The memory 130 is a non-transitory computer readable memory configured to store the electronic user record 140. The communication module 130 communicates the stored electronic user record 140 at the point of service. The stored electronic user record 140 may be accessed wirelessly, magnetically, or via a connection interface, such as a data port 170 as illustrated in FIG. 3. The stored electronic user record 140 may be accessed as a PDF or Word document.

The communication module 130 may comprise a wireless communicator 122, such as a radio frequency identification (RFID) antenna, a wireless tag, a near field communication (NFC) tag, Bluetooth^{®}, or any other similar contactless one or two way communication device configured to communicate the stored electronic user record wirelessly. The communication module 130 may alternatively comprise a magnetic strip 124 containing magnetic tracks to store the data. The information storage device 100 may also utilize a bar code or quick response (QR) code embossed or printed onto the device 100 to provide access to data.

The information storage device 100 may further comprise an encryption module 150. The encryption module 150 is configured to protect and limit access to the stored electronic user record 140 on the information storage device 100 via tokenization, encryption algorithms, personal identification numbers, and the like. The information storage device 100 may further comprise a biometric module 160. The biometric module 160 may be a fingerprint identifier scanner for limiting access to the stored electronic user record 140.

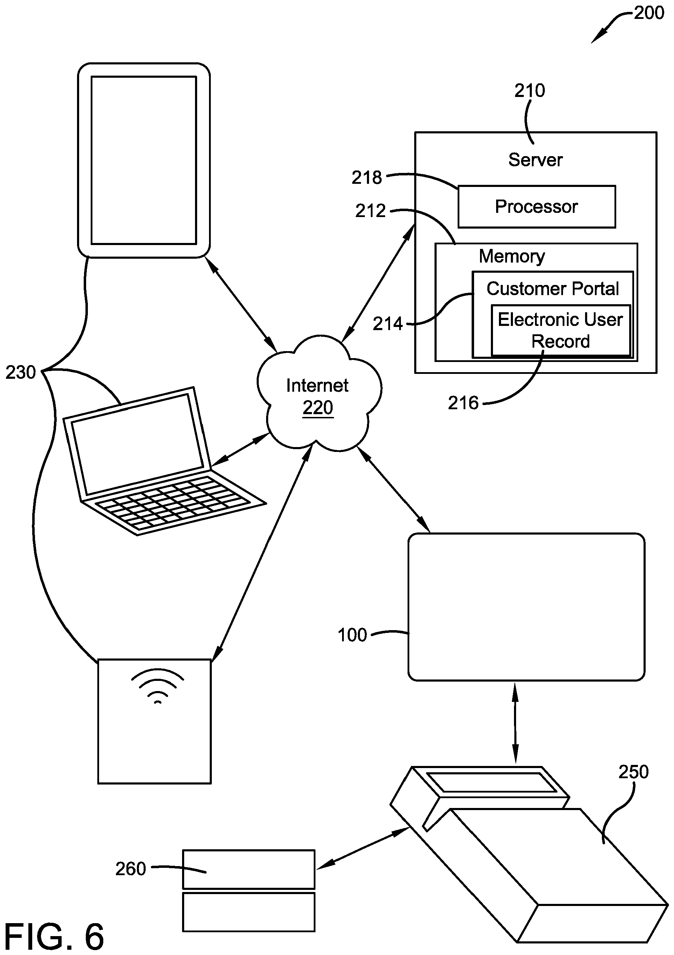

As illustrated in FIG. 6, the subject matter disclosed and claimed herein, in one embodiment thereof, comprises a system 200 for managing an electronic user record 216. The system 100 comprises a computer server 210, a plurality of portable information storage devices 100, and a plurality of external device interfaces 250. The system 200 is configured to allow the user to have their portable electronic user record 216 accessible at a point of service. The portable electronic user record 216 can be updated as required at the point of service or remotely on the computer server 210. The system 100 alleviates the need for filling out paperwork at a medical facility, an imaging provider, a laboratory, a medical provider's office, a dental office, a rehabilitation facility, a pharmacy, a therapist's office, a long term care facility, or any other location that may require the user's medical record or related demographic information prior to service.

The computer server 210 is a remote server comprising a memory 212 and a processor 218. The memory 212 stores a set of computer readable software instructions configured as a customer portal 214. The customer portal 214 is remotely accessible to the user over a network 220 via a user access device 230. The user access device 230 may be a smart phone, a laptop, a personal computer, a personal digital assistant, or any other remote device capable of accessing the internet.

The customer portal 214 is configured to receive a request from the user access device 230 to create the portable electronic user record 216. Once the portable electronic user record 216 is created, the customer portal 214 accepts an upload of the user's medical 142 and demographic data 144. The uploaded user's medical 142 and demographic data 144 is then updated to the electronic user record 216 hosted on the customer portal 216. The user may further update the electronic user record 216 over the network 220 via the user access device 230 as needed. The electronic user record 216 is then downloadable to one of the portable information storage devices 100.

As discussed supra, each information storage device 100 comprises a memory 130, a communication module 120, and a processor 110. The processor 110 is in communication with the memory 130 and communication module 120. The memory 130 is a non-transitory computer readable memory configured to store the electronic user record 140. The communication module 130 communicates the stored electronic user record 140 at a point of service. The stored electronic user record 140 may be accessed wirelessly, magnetically, or via a connection interface, such as a data port 170.

The communication module 130 may comprise a wireless communicator 122, such as a radio frequency identification (RFID) antenna, a wireless tag, a near field communication (NFC) tag, Bluetooth^{®}, or any other similar contactless one or two way communication device configured to communicate the stored electronic user record wirelessly. The communication module 130 may alternatively comprise a magnetic strip 124 containing magnetic tracks to store the data. The information storage device 100 may also utilize a bar code or quick response (QR) code embossed or printed onto the device to access data.

The information storage device 100 may further comprise an encryption module 150. The an encryption module 150 is configured to protect and limit access to the stored electronic user record 140 on the information storage device 100 via tokenization, encryption algorithms, personal identification numbers, and the like. The information storage device 100 may further comprise a biometric module 160. The biometric module 160 may be a fingerprint identifier scanner for limiting access to the stored electronic user record 140.

Each external device interface 250 is configured to communicate with each portable information storage device 100 and access the stored electronic user record 140 stored thereon. Each external device interface 250 may also be configured to communicate with or access an electronic medical record system 260 at the point of service and to communicate the stored electronic user record 140 to the electronic medical record system 260. The external device interface 250 is a reader or other scanning device, such as, but not limited to, a card reader, a RFID reader, an NFC tag reader, a magnetic code reader, a bar or QR code reader, or the like. The electronic medical record system 260 may be any electronic provider charting or billing system used in the medical industry. New medical or demographic data from the current visit may then be transferred from the electronic medical record system 260 to the portable information storage device 100 via the external device interface 260. Alternatively, the user or provider may update the electronic user record 216 over the network 220 via the user access device 230 which is then used to update the electronic user record 140 on the portable information storage device 100.

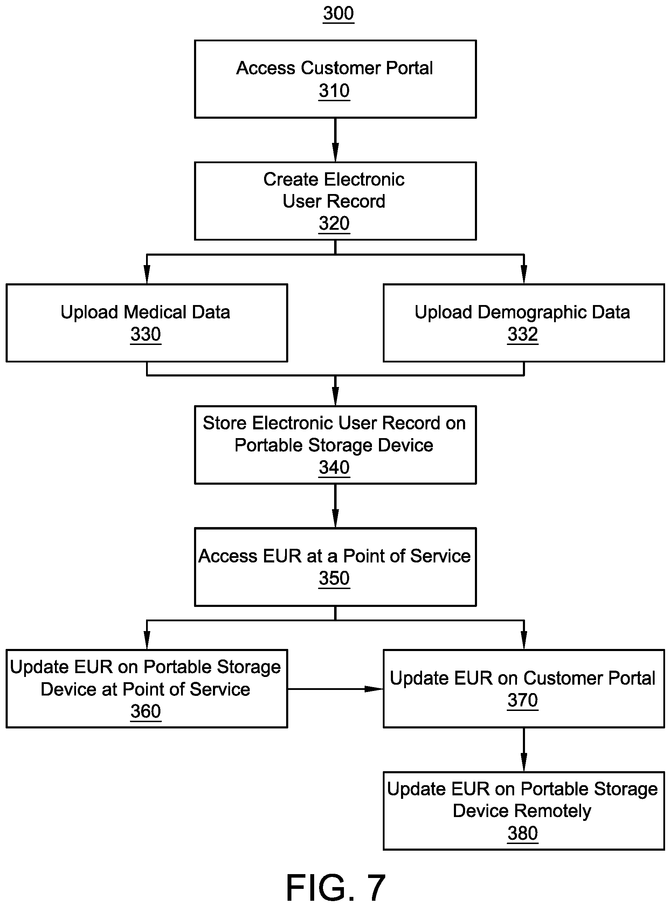

As illustrated in FIG. 7, the subject matter disclosed and claimed herein, in one embodiment thereof, comprises a method 300 for creating and administering a user's electronic user record 216. The user accesses a customer portal 214 stored on a computer-based system 200 over a network 220 using a user access device 230. The user then constructs the electronic user record 216 on the customer portal 214. The user uploads a user's medical data 142 and a user's demographic data 144 to the customer portal 214 into the electronic user record 216. The user's electronic user record 216 is then further stored on a portable information storage device 100 as discussed *supra.*

The user's electronic user record 140 on the portable information storage device 100 is then accessible at a point of service via an external device interface 250. The external device interface 250 can upload the electronic user record 140 to an electronic medical records system 260 of the point of service. Then, the external device interface 250 can modify the electronic user record 140 with any new user medical or demographic data. Alternatively, the user may update the electronic user record 216 via the customer portal 214 on the computer-based system 200. Then the updated electronic user record 216 is then updated on the portable information storage device 100.

It is contemplated that information storage device 100 constructed in accordance with the present invention will be tailored and adjusted by those of ordinary skill in the art to accommodate various levels of performance demand imparted during actual use. Accordingly, while this invention has been described by reference to certain specific embodiments and examples, it will be understood that this invention is capable of further modifications. This application is, therefore, intended to cover any variations, uses or adaptations of the invention following the general principles thereof, and including such departures from the present disclosure as come within known or customary practice in the art to which this invention pertains and fall within the limits of the appended claims.

Notwithstanding the foregoing, the information storage device 100 of the present invention and its various structural components can be of any suitable size, shape, and configuration as is known in the art without affecting the overall concept of the invention, provided that it accomplishes the above stated objectives. One of ordinary skill in the art will appreciate that the shape and size of information storage device 100 and its various components and material, as shown in the FIGS. are for illustrative purposes only, and that many other shapes and sizes of information storage device 100 are well within the scope of the present disclosure. Although the dimensions of information storage device 100 are important design parameters, information storage device 100 and its components may be of any shape or size that ensures optimal performance during use and/or that suits user need and/or preference.

What has been described above includes examples of the claimed subject matter. It is, of course, not possible to describe every conceivable combination of components or methodologies for purposes of describing the claimed subject matter, but one of ordinary skill in the art may recognize that many further combinations and permutations of the claimed subject matter are possible. Accordingly, the claimed subject matter is intended to embrace all such alterations, modifications and variations that fall within the spirit and scope of the appended claims. Furthermore, to the extent that the term "includes" is used in either the detailed description or the claims, such term is intended to be inclusive in a manner similar to the term "comprising" as "comprising" is interpreted when employed as a transitional word in a claim.

## Claims

1. An information storage device for retaining an electronic user record, the information storage device comprising:
a non-transitory computer readable memory for storing the electronic user record;
a communication module for communicating the stored electronic user record at a point of service; and
a processor in communication with the memory and the communication module; and
wherein the electronic user record comprises a user's medical data and a user's demographic data.

2. The information storage device of claim 1 further comprising an encryption module for limiting access to the stored electronic user record.

3. The information storage device of claim 1 or 2, wherein the communication module is configured to communicate the stored electronic user record wirelessly.

4. The information storage device of any preceding claim, wherein the information storage device is portable.

5. The information storage device of claim 4, wherein the information storage device is a magnetic strip card.

6. The information storage device of claim 4, wherein the information storage device is an integrated circuit card.

7. The information storage device of any preceding claim, wherein the electronic user record is updatable.

8. A system for managing an electronic user record, the system comprising:
a computer server comprising a memory storing a set of computer readable software instructions configured as a customer portal, that when executed by a processor, causes the server to:
access a request from a user access device to create an electronic user record;
upload a user's medical data to the electronic user record;
upload a user's demographic data to the electronic user record;
update the electronic user record; and
a plurality of portable information storage devices, each device comprising a non-transitory computer readable memory for storing the electronic user record, a communication module for receiving and communicating the stored electronic user record, and a processor in communication with the memory and the communication module; and
a plurality of external device interfaces each configured to access the electronic user record from each of the plurality of portable information storage devices.

9. The system of claim 8, wherein each of the information storage devices is an integrated circuit card.

10. The system of claim 8 or 9, wherein the communication module is configured to communicate with any of the plurality of external device interfaces wirelessly.

11. The system of any of claims 8 to 10, wherein each of the plurality of external device interfaces is further configured to access an electronic medical record system.

12. The system of any of claims 8 to 11, wherein each of the plurality of external device interfaces are further configured to update the electronic user record on each of the portable information storage devices.

13. A method for creating and administering an electronic user record on a computer-based system accessible storable on a portable information storage device connected to the computer-based system over a network, the method comprising:
constructing an electronic user record on a customer portal by receiving, by a processor of the computer-based system, from a processor of the user access device, a request from the user access device to create the electronic user record;
uploading a user's medical data to the customer portal;
uploading a user's demographic data to the customer portal;
storing the electronic user record on a portable information storage device;
accessing the electronic user record on the portable information storage device via an external device interface; and
updating the electronic user record on the customer portal and the portable information storage device.

14. The method of claim 13, wherein the portable information storage device comprises a non-transitory computer readable memory for storing an electronic user record, a communication module for communicating the stored electronic user record to the plurality of information systems, and a processor in communication with the memory and the communication module.

15. The method of claim 13 or 14, wherein the external device interface is configured to access an electronic medical record system and to update the electronic user record on the portable information storage device.
